(19) **Europäisches Patentamt / European Patent Office / Office européen des brevets**

(11) **EP 1 767 942 A1**

(12) **EUROPEAN PATENT APPLICATION**
published in accordance with Art. 158(3) EPC

(43) Date of publication:
**28.03.2007 Bulletin 2007/13**

(21) Application number: **05751289.9**

(22) Date of filing: **14.06.2005**

(51) Int Cl.:
*G01N 33/543* (2006.01)     *G01N 33/53* (2006.01)
*G01N 33/531* (2006.01)

(86) International application number:
**PCT/JP2005/010882**

(87) International publication number:
**WO 2005/121795 (22.12.2005 Gazette 2005/51)**

(84) Designated Contracting States:
**AT BE BG CH CY CZ DE DK EE ES FI FR GB GR HU IE IS IT LI LT LU MC NL PL PT RO SE SI SK TR**

(30) Priority: **14.06.2004 JP 2004176288**

(71) Applicant: **KYOWA MEDEX CO., LTD.**
**Chuo-ku, Tokyo 104-6004 (JP)**

(72) Inventors:
• **MORITA, Kazuki**
  C/O Kyowa Medex Res. Lab.
  Sunto-Gun, Shizuoka 411-0932 (JP)

• **UZAWA, Koji**
  c/o Kyowa Medex Res Lab.
  Sunto-gun, Shizuoka 411-0932 (JP)
• **SUZUKI, Emiko,**
  c/o Kyowa Medex Res. Lab.
  Sunto-Gun, Shizuoka 411-0932 (JP)

(74) Representative: **Vossius & Partner**
  **Siebertstrasse 4**
  **81675 München (DE)**

(54) **IMMUNOASSAY IN WHICH NON-SPECIFIC REACTION IS SUPPRESSED AND REAGENT THEREFORE**

(57) A method for immunologically determining an analyte in which a nonspecific reaction is suppressed, which comprises the steps of:
allowing an enzyme-labeled antibody in which an enzyme is bound as a label to a first antibody that specifically binds to the analyte, to react with the sample in an aqueous medium to form an immune complex of the analyte and the enzyme-labeled antibody, said enzyme-labeled antibody substantially containing only one or plural number of enzyme-labeled antibodies selected from the group consisting of enzyme-labeled antibodies having a binding ratio between the first antibody and the enzyme of 1:1, 1:2, 1:3 and 2:1, respectively, and measuring the enzyme activity of the immune complex.

Fig. 1

EP 1 767 942 A1

**Description**

TECHNICAL FIELD

The present invention relates to a method for immunologically determining an analyte in which a nonspecific reaction is suppressed, a reagent for use in the method for immunologically determining an analyte in which a nonspecific reaction is suppressed, and a method for suppressing a nonspecific reaction in a method for immunologically determining an analyte.

BACKGROUND ART

In methods for immunologically determining an analyte, occurrence of a nonspecific reaction has been often found. Although various kinds of the nonspecific reaction in antigen-antibody reactions have been known, in particular, nonspecific reactions caused by a human anti-mouse antibody (hereinafter, abbreviated as HAMA) have been problematic. There are two types of HAMA, i.e., HAMA type I and HAMA type II. The HAMA type I is appeared in blood of humans who have never sensitized by a mouse protein, while the HAMA type II is appeared in humans who have been in contact with mouse such as animal breeders, and humans who have been administered with a mouse protein such as a mouse antibody. Generally, HAMA is a nonspecific factor which can be problematic when a mouse antibody is used in a method for the immunological determination. It is known that HAMA causes an error in the measurement system to lead to failure in the measurement of an accurate value. Therefore, upon monitoring pathologic conditions and the like based on determination values for analytes which are obtained by an immunoassay method in which the analyte in a sample is determined, erroneous decision could be made. Accordingly, development of a measurement method which provides accurate determination values has been desired.

As a method for suppressing a nonspecific reaction by HAMA in the method for the immunological determination, it is known that immunoglobulin in the same animal species as that of the antibody used in the immunoassay, polymerized immunoglobulin and the like are effective (see, Patent Document 1, Patent Document 2, Nonpatent Document 1, Nonpatent Document 2 and Nonpatent Document 3).

Also, as another nonspecific reaction in antigen-antibody reactions, there is a nonspecific reaction caused by an antibody which reacts with the enzyme used for labeling the antibody, and thus a method for suppressing a nonspecific reaction in which the enzyme which had been inactivated is allowed to coexist in the measurement system was reported (see, Patent Document 3).

Interleukin-2 (hereinafter, referred to as IL-2) is a cytokine composed of 133 amino acids, which is produced predominantly from T cells such as $CD4^+$ or $CD8^+$, but is also produced from natural killer cells (NK cells). IL-2 primarily participates in activation of an immune system, and has various bioactivities. For example, IL-2 acts as a progression factor that promotes progression of the cell cycle on T cells, B cells, NK cells, LAK cells (lymphokine activated killer cells), macrophages, neutrophils and the like.

It is known that IL-2 receptors (hereinafter, referred to as IL-2R) are composed of $\alpha$ chain, $\beta$ chain and $\gamma$ chain, and a soluble interleukin-2 receptor (hereinafter, referred to as sIL-2R) in which a part of the $\alpha$ chain is released from the cell surface is preset in blood. It is reported that sIL-2R level in blood is elevated upon activation of living body immune defense mechanisms, and activation of T cell line and B cell line, because sIL-2R is produced by activated T cells and B cells. It is reported that high concentration of sIL-2R in serum is high in patients suffering from autoimmune diseases such as chronic rheumatoid arthritis and systemic lupus erythematosus (SLE) as well as viral infectious diseases such as viral hepatitis and acquired immune deficiency syndrome (AIDS), and it shall be the one of the marker of the activated lymphocyte in a living body. Furthermore, it is known that tumor cells produce sIL-2R, and thus progression of adult T cell leukemia (ATL) and non-Hodgkin's lymphoma correlates well with variation of the sIL-2R concentration in the serum. Accordingly, the correlation with various diseases and pathologic conditions of immune systems and is reported, hence it has become a promising marker in blood for hematopoietic diseases. The sIL-2R concentration in serum has been clinically utilized efficiently as a marker and the like for monitoring of pathologic condition in adult T cell leukemia, and as a marker and the like for evaluation of therapeutic effects, follow-up after the remission and early detection of recurrence in non-Hodgkin's lymphoma.

Hitherto, an immunological measurement method in which two anti-sIL-2R antibodies are used has been reported (see, Patent Document 4) as the measurement method of sIL-2R, and as a reagent for measuring sIL-2R, reagents suited for the enzyme immunoassay methods, for example, "CELLFREE IL-2R" (manufactured by Yamanouchi Pharmaceutical Co., Ltd.) and the like have been developed and have been on the market.

Patent Document 1: Japanese Published Patent Application No. 65162/86
Patent Document 2: Japanese Published Patent Application No. 254869/89
Patent Document 3: Japanese Published Patent Application No. 188055/93
Patent Document 4: Japanese Published Patent Application No. 70761/87

Nonpatent Document 1: Clinical Chemistry, 1999, Vol. 45, pp. 942-956
Nonpatent Document 2: Cancer Immunological Immunotherapy, 1991, Vol. 33, pp. 80-84
Nonpatent Document 3: Clinical Chemistry, 1990, Vol. 36, p. 1093

DISCLOSURE OF THE PRESENT INVENTION

Problems to be solved by the present invention

An object of the present invention is to provide a method for immunologically determining an analyte in which a nonspecific reaction is suppressed, a reagent for use in the method for immunologically determining an analyte in which a nonspecific reaction is suppressed, and a method for suppressing a nonspecific reaction in the method for immunologically determining an analyte.

Means for Solving the Problems

The present invention relates to the following (1) to (35).

(1) A method for, immunologically determining an analyte in a sample, in which a non-specific reaction is suppressed, which comprises the steps of:

allowing an enzyme-labeled antibody in which an enzyme is bound as a label to a first antibody that specifically binds to the analyte, to react with the sample in an aqueous medium thereby forming an immune complex of the analyte and the enzyme-labeled antibody, said enzyme-labeled antibody substantially containing only one or plural number of enzyme-labeled antibodies selected from the group consisting of enzyme-labeled antibodies having a binding ratio between the first antibody and the enzyme of 1:1, 1:2, 1:3 and 2:1, respectively, and measuring the enzyme activity of the immune complex.

(2) The method for immunologically determining an analyte according to (1) which further comprises the step of allowing an antibody in which a separation means is bound to a second antibody that specifically binds to the antigenic determination site that is different from the antigenic determination site of the analyte to which the first antibody is bound to react with the sample, thereby forming an immune complex with the analyte.
(3) The method for immunologically determining an analyte according to (1) or (2) wherein the binding ratio between the first antibody and the enzyme is 1:1, 1:2 and 1:3, respectively.

(4) The method for immunologically determining an analyte according to (1) or (2) wherein the binding ratio between the first antibody and the enzyme is 1:1 and 1:2, respectively.
(5) The method for immunologically determining an analyte according to any one of (1) to (4) wherein the first antibody is an antibody in which the Fc region is removed.
(6) The method for the immunological determination according to any one of (1) to (5) wherein the nonspecific reaction is a reaction that results from a human anti-mouse antibody.

(7) The method for immunologically determining an analyte according to any one of (1) to (6) wherein an IgG polymer and/or IgG are/is allowed to coexist in the step of allowing the enzyme-labeled antibody to react with the sample, thereby forming the immune complex with the analyte.
(8) The method for immunologically determining an analyte according to any one of (1) to (5) wherein the nonspecific reaction is a reaction that results from an antibody that reacts with the labeling enzyme.

(9) The method for immunologically determining an analyte according to any one of (1) to (8) wherein an inactivated enzyme which is the same enzyme as the enzyme labeling the antibody is allowed to coexist in the step of allowing the enzyme-labeled antibody to react with the sample, thereby forming the immune complex with the analyte.

(10) The method for immunologically determining an analyte according to any one of (1) to (9) wherein the enzyme is peroxidase.

(11) The method for immunologically determining an analyte according to any one of (1) to (10) wherein the analyte is a soluble interleukin-2 receptor.

(12) A reagent for immunologically determining an analyte in which a nonspecific reaction is suppressed, which comprises an enzyme-labeled antibody in which an enzyme is bound as a label to a first antibody that specifically binds to the analyte, said enzyme-labeled antibody substantially containing only one or plural number of enzyme-labeled antibodies selected from the group consisting of enzyme-labeled antibodies having a binding ratio between the first antibody and the enzyme of 1:1, 1:2, 1:3 and 2:1, respectively.

(13) The reagent according to (12) which further includes an antibody in which a separation means is bound to a second antibody that specifically binds to the antigenic determination site that is different from the antigenic determination site of the analyte to which the first antibody is bound.

(14) The reagent according to (12) or (13) which further comprises a reagent for measuring the enzyme activity.

(15) The reagent according to any one of (12) to (14) wherein the binding ratio between the first antibody and the enzyme is 1:1, 1:2 and 1:3, respectively.

(16) The reagent according to any one of (12) to (14) wherein the binding ratio between the first antibody and the enzyme is 1:1 and 1:2, respectively.

(17) The reagent according to any one of (12) to (16) wherein the first antibody is an antibody in which the Fc region is removed.

(18) The reagent according to any one of (12) to (17) wherein the nonspecific reaction is a reaction that results from a human anti-mouse antibody.

(19) The reagent according to any one of (12) to (18) which further comprises an IgG polymer and/or IgG.

(20) The reagent according to any one of (12) to (17) wherein the nonspecific reaction is a reaction that results from an antibody that reacts with the labeling enzyme.

(21) The reagent according to any one of (12) to (20) which further comprises an inactivated enzyme which is the same enzyme as the enzyme labeling the antibody.

(22) The reagent according to any one of (12) to (21) wherein the enzyme is peroxidase.

(23) The reagent according to any one of (12) to (22) wherein the analyte is a soluble interleukin-2 receptor.

(24) The reagent according to any one of (12) to (23) which further comprises one or plural number of substances selected from the group consisting of an aqueous medium, a metal ion, a salt, saccharide, a surfactant, an antiseptic agent, protein and a protein stabilizing agent.

(25) A method for suppressing a nonspecific reaction in a method for immunologically determining an analyte, which comprises the step of:

allowing an enzyme-labeled antibody in which an enzyme is bound as a label to a first antibody that specifically binds to the analyte, to react with the sample in an aqueous medium thereby forming an immune complex of the analyte and the enzyme-labeled antibody, said enzyme-labeled antibody substantially containing only one or plural number of enzyme-labeled antibodies selected from the group consisting of enzyme-labeled antibodies having a binding ratio between the first antibody and the enzyme of 1:1, 1:2, 1:3 and 2:1, respectively.

(26) The method for suppressing a nonspecific reaction according to (25) which further comprises the step of:

allowing an antibody in which a separation means is bound to a second antibody that specifically binds to the antigenic determination site that is different from the antigenic determination site of the analyte to which the first antibody is bound to react with the sample, thereby forming an immune complex with the analyte.

(27) The method for the suppression according to (25) or (26) wherein the binding ratio between the first antibody and the enzyme is 1:1, 1:2 and 1:3, respectively.

(28) The method for suppressing a nonspecific reaction according to (25) or (26) wherein the binding ratio between the first antibody and the enzyme is 1:1 and 1:2, respectively.
(29) The method for suppression a nonspecific reaction according to any one of (25) to (28) wherein the first antibody is an antibody in which the Fc region is removed.
(30) The method for suppression a nonspecific reaction according to any one of (25) to (29) wherein the nonspecific reaction is a reaction that results from a human anti-mouse antibody.
(31) The method for suppressing a nonspecific reaction according to any one of (25) to (30) wherein an IgG polymer and/or IgG are/is allowed to coexist in the step of allowing the enzyme-labeled antibody to react with the sample, thereby forming the immune complex with the analyte.

(32) The method for suppressing a nonspecific reaction according to any one of (25) to (29) wherein the nonspecific reaction is a reaction that results from an antibody that reacts with the labeling enzyme.
(33) The method for suppressing a nonspecific reaction according to any one of (25) to (32) wherein an in activated enzyme which is the same enzyme as the enzyme labeling the antibody is allowed to coexist in the step of allowing the enzyme-labeled antibody to react with the sample, thereby forming the immune complex with the analyte.
(34) The method for suppressing a nonspecific reaction according to any one of (25) to (33) wherein the enzyme is peroxidase.
(35) The method for suppressing a nonspecific reaction according to any one of (25) to (34) wherein the analyte is a soluble interleukin-2 receptor.

Effect of the present invention

According to the present invention, a method for immunologically determining an analyte in which a nonspecific reaction is suppressed, a reagent for immunologically determining an analyte in which a nonspecific reaction is suppressed, and a method for suppressing a nonspecific reaction in the method for immunologically determining an analyte are provided.

BRIEF DESCRIPTION OF THE DRAWINGS

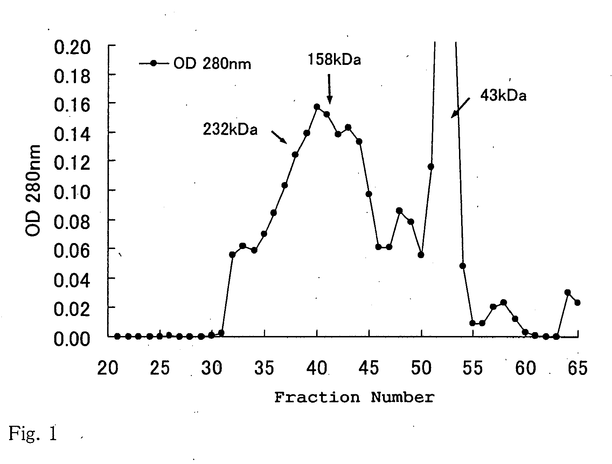

Fig. 1 shows a gel filtration column chromatogram of a POD-labeled anti-sIL-2R monoclonal F(ab')$_2$ antibody.

BEST MODE FOR CARRYING OUT THE PRESENT INVENTION

1. Nonspecific Reaction

The nonspecific reaction in the present invention is not particularly limited as long as it is a nonspecific reaction found in immunological measurement methods of an analyte, and examples thereof include nonspecific reactions resulting from HAMA, nonspecific reactions resulting from an antibody that reacts with the labeling enzyme, and the like. Examples of HAMA include HAMA type I and HAMA type II. Examples of the antibody that reacts with the labeling enzyme include e.g., antibodies that react with peroxidase, antibodies that react with alkaline phosphatase, and the like. The antibody that reacts with the labeling enzyme in the present invention is an antibody that reacts with the enzyme in the enzyme-labeled antibody used in the immunological measurement method of the present invention, and the antibody causes a nonspecific reaction.

## 2. Sample

Examples of the sample used in the present invention include e.g., whole blood, plasma, serum, spinal fluid, saliva, amniotic fluid, urine, sweat, pancreatic fluid and the like, and whole blood, plasma, serum and the like are preferred.

## 3. Analyte

The analyte of the present invention is not particularly limited and may be any analyte as long as it is a substance which can be an antigen, but is preferably an antigen having at least two antigenic determinants. Examples thereof include e.g., sIL-2R, myoglobin known as a marker for myocardial infarction, creatine kinase MB (CK-MB), troponin T, and the like.

## 4. Antibody

The first antibody for forming the enzyme-labeled antibody used in the present invention is not particularly limited as long as it is an antibody that specifically binds to the analyte, and any one of polyclonal antibodies and monoclonal antibodies can be used, but monoclonal antibodies are preferred. Moreover, in the present invention, not only the antibody but also an antibody fragment prepared by removing the Fc region such as Fab obtained by subjecting an antibody to a treatment with papain, $F(ab')_2$ obtained by a treatment with pepsin, and Fab' obtained by a treatment with pepsin-reduction treatment can be used. As the antibody fragment, $F(ab')_2$ is particularly preferable.

The second antibody for forming the solid phased antibody used in the present invention is not particularly limited as long as it is an antibody that specifically binds to the analyte, and any one of polyclonal antibodies and monoclonal antibodies can be used, but monoclonal antibodies are preferred. It is preferred that the second antibody is an antibody that specifically binds to an antigenic determination site that is different from the antigenic determination site of the analyte to which the first antibody is bound.

The antibody used in the present invention can be obtained by a common method using the analyte or a peptide that corresponds to its epitope as the antigen, but is also available as a commercial product. Examples of the antibody that specifically binds to sIL-2R include e.g., a monoclonal antibody produced by hybridoma AM92.3 (manufactured by PIERCE Inc.), a monoclonal antibody 7G7/B6 (manufactured by PIERCE Inc.; see, Japanese Published Patent Application No. 70761/87) and the like. Each can be arbitrarily used as the first antibody or the second antibody.

## 5. Enzyme Labeling

Examples of the enzyme that binds to the first antibody in the present invention include e.g., peroxidase (hereinafter, abbreviated as POD), alkaline phosphatase, luciferase, β-D-galactosidase, glucose oxidase and the like. Peroxidase and alkaline phosphatase are preferred, and peroxidase is particularly preferred. As the peroxidase, one of any origin can be used, but peroxidase derived from horseradish is preferred. Examples of the alkaline phosphatase include e.g., alkaline phosphatase derived from bovine small intestine, and the like.

## 6. Binding of First Antibody and Enzyme

The enzyme-labeled antibody in the present invention is one in which the enzyme is bound as the label to the first antibody that specifically binds to the analyte. Mode of binding in this bond may be for example, a covalent bond. The enzyme and the antibody may be either directly bound, or indirectly bound via a linker or the like. Examples of the method for producing the enzyme-labeled antibody include e.g., a glutaraldehyde method, a periodic acid method, a maleimide method, a pyridyl disulfide method and the like (for example, see "Enzyme Immunoassay Method", 1987, author: Eiji Ishikawa, published by Igaku-Shoin Ltd.), but the maleimide method is preferred. Specifically, for example, it can be prepare by mixing the antibody sulfhydrylated with iminothiolane or the like with the enzyme maleimidated with succinimidyl 4-[N-maleimidomethyl]-cyclohexane-1-carboxylate (SMCC), N-(6-maleimidocaproyloxy)succinimide (EMCS) or the like.

## 7. Binding ratio between Antibody and Enzyme in the Enzyme-Labeled Antibody

The enzyme-labeled antibody used in the present invention is an enzyme-labeled antibody in which a small number of labeling enzyme molecules is bound per molecule of the antibody. Specifically, it may be an enzyme-labeled antibody substantially containing only one or plural number of enzyme-labeled antibodies selected from the group consisting of enzyme-labeled antibodies having a binding rario between the first antibody and the enzyme of 1:1, 1:2, 1:3 and 2:1, respectively, preferably, an enzyme-labeled antibody substantially containing only one or plural number of enzyme-labeled antibodies selected from the group consisting of enzyme-labeled antibodies having a binding ratio between the first antibody and the enzyme of 1:1, 1:2 and 1:3, respectively, and more preferably, an enzyme-labeled antibody substantially containing only one or plural number of enzyme-labeled antibodies selected from the group consisting of enzyme-labeled antibodies having a binding ratio between the first antibody and the enzyme of 1:1 and 1:2, respectively.

The labeled antibody is particularly preferably the enzyme-labeled antibody having a binding ratio between the first antibody and the enzyme of 1:1 or 1:2. When the enzyme-labeled antibody is a mixture, proportion of the number of molecules of the enzyme-labeled antibody having a binding ratio between the first antibody and the enzyme of 1:1 or 1:2 to the number of molecules of the entire labeled antibodies is preferably 50% or greater, more preferably 70% or greater, particularly preferably 90% or greater.

Such an enzyme-labeled antibody can be prepared, after allowing the first antibody to bind to the enzyme by the aforementioned glutaraldehyde method, periodic acid method, maleimide method, pyridyl disulfide method or the like, by removing the first antibody to which a large number of molecules of the enzyme are bound, and unreacted enzyme and antibodies, using, for example, a method such as an ion exchanging chromatography method, a gel filtration column chromatography method, a hydrophobic chromatography method or the like, or any combination of the same, or the like.

8. Solid Phased Antibody

In the solid phased antibody according to the present invention, a separation means is bound to the second antibody that specifically binds to the analyte. As the separation means, a solid phase itself, or a substance which specifically binds to the substance bound to a solid phase, and the like may be exemplified. The mode of binding in such a bond may be a noncovalent bond when a solid phase is used, and may be a covalent bond when the substance that specifically binds to the substance bound to the solid phase is used, in which the antibody may be either directly bound to the substance or indirectly bound via a linker or the like. Examples of combination of the substance bound to the solid phase, and the substance that specifically binds thereto include a combination of biotin and avidin, and the like.

The solid phase is not particularly limited as long as it is a solid phase which immobilizes the second antibody and which enables the immunological measurement method of the analyte, and examples thereof include e.g., polystyrene plates such as microtiter plates, particulate matters (beads) made of glass or a synthetic resin, spherical matters (balls) made of glass or a synthetic resin, latex, magnetic particles, a various membranes such as nitrocellulose membranes, test tubes made of a synthetic resin, and the like.

9. IgG Polymer and IgG

The IgG polymer in the present invention is not particularly limited as long as it is formed by polymerization of IgG, and examples thereof include e.g., MAK33-IgG1/IgG1 Poly, MAK33-IgG(2b)/Fab(2a) Poly (both manufactured by Roche Diagnostics), and the like. IgG in the present invention is not particularly limited as long as it is an animal IgG, and examples thereof include e.g., IgG of animals such as mouse, rat, hamster, rabbit, guinea pig, goat, sheep, chicken, cattle, horse and the like. Among these, mouse IgG is preferred. The animal IgG may be either purified one, or a serum of the animal.

10. Aqueous Medium

Examples of the aqueous medium include e.g., deionized water, distilled water, buffers and the like, and the buffers are preferred. The buffer for use in the preparation of the buffer is not particularly limited as long as it has a buffer action, and examples thereof include ones having a pH of 1 to 11, e.g., a lactate buffer, a citrate buffer, an acetate buffer, a succinate buffer, a phtharate buffer, a phosphate buffer, a triethanolamine buffer, a diethanolamine buffer, a lysine buffer, a barbiturate buffer, an imidazole buffer, a malate buffer, an oxalate buffer, a glycine buffer, a borate buffer, a carbonate buffer, a glycine buffer, a Good's buffer, and the like.

Examples of the Good's buffer include e.g., a 2-morpholinoethanesulfonic acid (MES) buffer, a bis(2-hydroxyethyl)iminotris(hydroxymethyl)methane (Bis-Tris) buffer, a tris(hydroxymethyl)aminomethane (Tris) buffer, an N-(2-acetoamide)imino diacetic acid (ADA) buffer, a piperazine-N,N'-bis(2-ethanesulfonic acid) (PIPES) buffer, a 2-[N-(2-acetoamide)amino]ethanesulfonic acid (ACES) buffer, a 3-morpholino-2-hydroxypropanesulfonic acid (MOPSO) buffer, a 2-[N,N-bis(2-hydroxyethyl)amino]ethanesulfonic acid (BES) buffer, a 3-morpholinopropanesulfonic acid (MOPS) buffer, a 2-{N-[tris(hydroxymethyl)methyl]amino}ethanesulfonic acid (TES) buffer, an N-(2-hydroxyethyl)-N'-(2-sulfoethyl)piperazine (HEPES) buffer, a 3-[N,N-bis(2-hydroxyethyl)amino]-2-hydroxypropanesulfonic acid (DIPSO) buffer, a 2-hydroxy-3-{[N-tris(hydroxymethyl)methyl]amino}propanesulfonic acid (TAPSO) buffer, a piperazine-N,N'-bis(2-hydroxypropane-3-sulfonic acid) (POPSO) buffer, an N-(2-hydroxyethyl)-N'-(2-hydroxy-3-sulfopropyl)piperazine (HEPPSO) buffer, an N-(2-hydroxyethyl)-N'-(3-sulfopropyl)piperazine (EPPS) buffer, a tricine[N-tris(hydroxymethyl)methylglycine] buffer, a vicine[N,N-bis(2-hydroxyethyl)glycine] buffer, a 3-[N-tris(hydroxymethyl)methyl]aminopropanesulfonic acid (TAPS) buffer, a 2-(N-cyclohexylamino)ethanesulfonic acid (CHES) buffer, a 3-(N-cyclohexylamino)-2-hydroxypropanesulfonic acid (CAPSO) buffer, a 3-(N-cyclohexylamino)propanesulfonic acid (CAPS) buffer, and the like.

The concentration of the buffer is not particularly limited as long as it is a concentration suitable for the measurement, but is preferably 0.001 to 2.0 mol/L, more preferably 0.005 to 1.0 mol/L, and particularly preferably 0.01 to 0.1 mol/L.

11. Metal Ion

Examples of the metal ion include e.g., magnesium ion, manganese ion, zinc ion, and the like.

12. Salts

Examples of the salt include e.g., sodium chloride, potassium chloride, and the like.

13. Saccharide

Examples of the saccharide include e.g., mannitol, sorbitol, and the like.

14. Surfactant

Examples of the surfactant include e.g., a nonionic surfactant, a cationic surfactant, an anionic surfactant, an amphoteric surfactant and the like, and an nonionic surfactant is preferred. Examples of the nonionic surfactant include e.g., Tween 20, Nonidet P-40 (NP-40), and the like.

15. Antiseptic Agent

Examples of the antiseptic agent include e.g., sodium azide, antibiotics (streptomycin, penicillin, gentamicin and the like), Bioace, Proclin 300, Proxel GXL, and the like.

16. Protein

Examples of the protein include e.g., bovine serum albumin (BSA), fetal bovine serum (FBS), casein, blockAce™ (manufactured by Dai Nippon Pharmaceutical Co., Ltd.), and the like.

17. Protein Stabilizing Agent

Examples of the protein stabilizing agent include e.g., Peroxidase Stabilizing Buffer (manufactured by DakoCytomation), and the like.

18. Inactivated Enzyme

The inactivated enzyme in the invention the present invention is not particularly limited as long as it is an inactivated enzyme that is the same enzyme as the enzyme labeling antibody for use in the method for immunologically determining the analyte of the present invention. In other words, the enzyme in the inactivated enzyme is derived from the same organism and has the same enzyme activity as to the enzyme in the enzyme-labeled antibody. For example, when a POD-labeled antibody is used in the method for immunologically determining an analyte, an inactivated enzyme obtained by inactivating the POD may be used, while when an alkaline phosphatase labeled antibody is used, an inactivated enzyme obtained by inactivating the alkaline phosphatase may be used. Although the inactivated enzyme may be bound to a carrier or an antibody, the antibody in this case must be one not to react with the analyte and the antibody. The term inactivation means to completely or substantially lose the enzyme activity which participates in the method for the immunological determination of the present invention while keeping the reactivity to the antibody that reacts with the labeled antibody which may result in the nonspecific reaction, and the inactivation can be carried out by a heat treatment, a denaturation treatment with an acid or an alkali, enzymatic digestion by protease, a treatment such as freezing and thawing, or any combined treatment of the same. For example, in the case of POD, the inactivated POD can be obtained by a treatment at 100 to 125°C for 10 to 60 minutes. Examples of the inactivated POD include Inactive Poly-POD (manufactured by Roche Diagnostics). Examples of the inactivated alkaline phosphatase include Scavenger-ALP (manufactured by Oriental Yeast Co., Ltd.) and the like.

19. Method for the Immunological Determination of Analyte

The method for immunologically determining an analyte of the present invention is not particularly limited as long as it is a method comprising the step of allowing an enzyme-labeled antibody in which an enzyme is bound as a label to a first antibody that specifically binds to the analyte, to react with the sample thereby forming an imune complex of the analyte and the enzyme-labeled antibody, said enzyme-labeled antibody substantially containing only one or plural number of enzyme-labeled antibodies selected from the group consisting of enzyme-labeled antibodies having a binding ratio between the first antibody and the enzyme of 1:1, 1:2, 1:3 and 2:1, respectively, and the step of measuring the enzyme activity of the immune complex. Furthermore, a method further comprising the step of allowing an antibody in which a separation means is bound to a second antibody that specifically binds to an antigenic determination site that is different from the antigenic determination site of the analyte to which the first antibody is bound to react with the sample, thereby forming an immune complex with the analyte is preferred.

The enzyme-labeled antibody is preferably an enzyme-labeled antibody substantially containing only one or plural number of enzyme-labeled antibodies selected from the group consisting of enzyme-labeled antibodies having a binding ratio between the first antibody and the enzyme of 1:1, 1:2 and 1:3, respectively, and more preferably an enzyme-labeled antibody substantially containing only one or plural number of enzyme-labeled antibodies selected from the group consisting of enzyme-labeled antibodies having a binding ratio between the first antibody and the enzyme of 1:1 and 1:2, respectively.

As the labeled antibody, an enzyme-labeled antibody having a binding ratio between the first antibody and the enzyme of 1:1 or 1:2 is particularly preferable. When the enzyme-labeled antibody is a mixture, proportion of the number of molecules of the enzyme-labeled antibody having a binding number between the first antibody and the enzyme of 1:1 or 1:2 to the number of molecules of entire labeled antibodies is preferably 50% or greater, more preferably 70% or greater, particularly preferably 90% or greater.
Specific examples of the method for determining an analyte include e.g., sandwich methods, competition methods and the like, and the sandwich methods are preferred. Examples of the sandwich method include e.g., one step method, delay one step method, two step method and the like.

More specific examples of the method for immunologically determining the analyte include e.g., a methods comprising the following steps:

(1) a step of allowing the solid phased antibody to react with the analyte in the sample, thereby forming an immune complex (primary reaction),
(2) a step of allowing the enzyme-labeled antibody to react with the analyte in the sample, thereby forming an immune complex (secondary reaction),
(3) a step of separating the enzyme-labeled antibody which does not form the immune complex from the solid phase,
(4) a step of measuring the enzyme activity of the immune complex formed on the solid phase,
(5) a step of quantitatively determining the analyte by comparing the measured enzyme activity in the step (4) based on the calibration curve prepared using the analyte having a previously known concentration.

A step of washing the solid phase following the primary reaction may be added as needed between the above step (1) and step (2). Also, the step (1) and the step (2) may be concurrently carried out.
It is preferred that the primary reaction be carried out in an aqueous medium. Reaction temperature of the primary reaction may be, for example, 0 to 50°C, preferably 4°C to 40°C. Reaction time of the primary reaction may be, for example, 5 minutes to 20 hrs. Examples of the washing solution used in washing the solid phase after the primary reaction include e.g., phosphate buffered physiological saline (10 mmol/L phosphate buffer, pH 7.2, containing 0.15 mol/L sodium chloride, hereinafter, referred to as PBS), PBS containing a surfactant, and the like. Examples of the surfactant include e.g., nonionic surfactants such as Tween 20.

It is preferred that the secondary reaction be carried out in an aqueous medium. It is preferred that antigenic determinant in the antibody (first antibody) that is used in the secondary reaction to form the enzyme-labeled antibody be different from the antigenic determinant in the antibody (second antibody) used in the primary reaction. Reaction temperature of the secondary reaction may be, for example, 0 to 50°C, preferably 4°C to 40°C. Reaction time of the secondary reaction may be, for example, 5 minutes to 20 hrs. Examples of the wash liquid used in washing the solid phase after the primary reaction include e.g., the wash liquids described above, and the like.

In the method for measuring the activity of the enzyme label in the immune complex formed on the solid phase by the secondary reaction, the enzyme activity in the immune complex can be measured by allowing a substrate for the enzyme to react with the enzyme, and measuring the produced substance. The reaction of the enzyme with the substrate for the enzyme is preferably carried out in an aqueous medium.

When the enzyme is peroxidase, peroxidase activity in the immune complex can be measured by, for example, an absorbency method, a fluorescence method, a luminescence method or the like. The methods for measuring the peroxidase activity by the absorbency method may be e.g., methods in which a combination of peroxidase, hydrogen peroxide that is the substrate therefor and an oxidative coloring chromogen is allowed to react, and the absorbance of the reaction liquid is measured by a spectrophotometer or the like. Examples of the oxidative coloring chromogen include e.g., leuco type chromogen, oxidative coupling-coloring chromogen and the like.

The leuco type chromogen is a substance converted alone into a dye in the presence of hydrogen peroxide and a peroxidative active substance such as peroxidase. Specific examples thereof include O-phenylenediamine (OPD), tetramethylbenzidine (TMB), 10-N-carboxymethylcarbamoyl-3,7-bis(dimethylamino)-10H-phenothiazine (CCAP), 10-N-methylcarbamoyl-3,7-bis(dimethylamino)-10H-phenothiazine    (MCDP),    N-(carboxymethylaminocarbonyl)-4,4'-bis (dimethylamino)diphenylamine sodium salt (DA-64), 4,4'-bis(dimethylamino)diphenylamine, bis[3-bis(4-chlorophenyl) methyl-4-dimethylaminophenyl]amine (BCMA) and the like.

The oxidative coupling-coloring chromogen is a substance that produces a dye through oxidative coupling of two compounds in the presence of hydrogen peroxide and a peroxidative active substance such as peroxidase. Examples of combination of the two compounds include combinations of a coupler and an aniline (Trinder reagent), combinations of a coupler and a phenol, and the like. Examples of the coupler include e.g., 4-aminoantipyrine (4-AA), 3-methyl-2-benzothiazolinonehydrazine and the like. Examples of the anilines include N-(3-sulfopropyl)aniline, N-ethyl-N-(2-hydroxy-3-sulfopropyl)-3-methylaniline (TOOS), N-ethyl-N-(2-hydroxy-3-sulfopropyl)-3,5-dimethylaniline (MAOS), N-ethyl-N-(2-hydroxy-3-sulfopropyl)-3,5-dimethoxyaniline (DAOS), N-ethyl-N-(3-sulfopropyl)-3-methylaniline (TOPS), N-(2-hydroxy-3-sulfopropyl)-3,5-dimethoxyaniline    (HDAOS),    N,N-dimethyl-3-methylaniline,    N,N-di(3-sulfopropyl)-3,5-dimethoxyaniline,    N-ethyl-N-(3-sulfopropyl)-3-methoxyaniline,    N-ethyl-N-(3-sulfopropyl)aniline,    N-ethyl-N-(3-sulfopropyl)-3,5-dimethoxyaniline, N-(3-sulfopropyl)-3,5-dimethoxyaniline, N-ethyl-N-(3-sulfopropyl)-3,5-dimethylaniline, N-ethyl-N-(2-hydroxy-3-sulfopropyl)-3-methoxyaniline,    N-ethyl-N-(2-hydroxy-3-sulfopropyl)aniline,    N-ethyl-N-(3-methylphenyl)-N'-succinylethylenediamine (EMSE), N-ethyl-N-(3-methylphenyl)-N'-acetylethylenediamine, N-ethyl-N-(2-hydroxy-3-sulfopropyl)-4-fluoro-3,5-dimethoxyaniline (F-DAOS) and the like. Examples of the phenols include phenol, 4-chlorophenol, 3-methylphenol, 3-hydroxy-2,4,6-triiodobenzoic acid (HTIB), and the like.

In the measurement of hydrogen peroxide, the concentration of the peroxidative active substance is not particularly limited as long as it is a concentration suited for the measurement, but is preferably 1 to 100 kU/L when peroxidase is used as the peroxidative active substance. In addition, the concentration of the oxidative coloring chromogen is not particularly limited as long as it is a concentration suited for the measurement, but is preferably 0.01 to 10 g/L.

Examples of the method for measuring the peroxidase activity by the fluorescence method include e.g., methods in which a combination of peroxidase, hydrogen peroxide that is the substrate therefor and a fluorescent substance is allowed to react, and intensity of the generated fluorescence is measured, and the like. Examples of the fluorescent substance include e.g., 4-hydroxyphenylacetic acid, 3-(4-hydroxyphenyl)propionic acid, coumarin and the like.

Examples of the method for the measurement of the peroxidase activity by the luminescence method include e.g., methods in which a combination of peroxidase, hydrogen peroxide that is the substrate therefor and a luminescent substance is allowed to react, and intensity of the generated luminescence is measured, and the like. Examples of the luminescent substance include e.g., luminol and the like.

When the enzyme is alkaline phosphatase, the alkaline phosphatase activity in the complex can be measured by, for example, a luminescence method or the like. Exemplary methods for measuring the alkaline phosphatase activity by the luminescence method are e.g., methods for the measurement in which alkaline phosphatase and a substrate therefor are allowed to react, and luminescent intensity of the generated luminescence is measured by a luminescence intensity meter or the like, and the like. Examples of the substrate for the alkaline phosphatase include e.g., 3-(2'-spiroadamantane)-4-methoxy-4-(3'-phosphoryloxy)phenyl-1,2-dioxetane disodium salt (AMPPD), 2-chloro-5-{4-methoxyspiro[1,2-dioxetane-3,2'-(5'-chloro)tricyclo(3.3.1.1$^{3,7}$)decane]-4-yl}phenylphosphate disodium salt (CDP-Star™), 3-{4-methoxyspiro [1,2-dioxetane-3,2'-(5'-chloro)tricyclo(3.3.1.1$^{3,7}$)decane]-4-yl}phenylphosphate disodium salt (CSPD™), [10-methyl-9 (10H)-acridinylidene]phenoxymethylphosphate disodium salt (Lumigen™ APS-5) and the like.

When the enzyme is luciferase, the luciferase activity in the immune complex can be measured by, for example, a luminescence method or the like. Exemplary methods for measuring the luciferase activity by the luminescence method are, for example, methods for the measurement in which luciferase and a substrate therefor are allowed to react, and luminescent intensity of the generated luminescence is measured by a luminescence intensity meter or the like, and the like. Examples of the substrate for luciferase include e.g., luciferin and the like.

When the enzyme is β-D-galactosidase, the β-D-galactosidase activity in the immune complex can be measured by, for example, an absorbency method (colorimetric method) or the like. Exemplary methods for measuring the β-D-galactosidase activity by the absorbency method (colorimetric method) are, for example, methods for the measurement in which β-D-galactosidase and a substrate therefor are allowed to react, and absorbance of the reaction liquid is measured by a spectrophotometer or the like, and the like. Examples of the substrate for the β-D-galactosidase include e.g., 2-chloro-4-nitrophenyl β-D-lactoside, 2-nitrophenyl-β-D-galactoside (ONPG), 5-bromo-4-chloro-3-indolyl-β-D-galactoside (X-gal) and the like.

When the enzyme is glucose oxidase, the glucose oxidase activity in the immune complex can be measured by allowing glucose that is the substrate for glucose oxidase to act on glucose oxidase, and measuring the produced hydrogen peroxide. The measurement of hydrogen peroxide can be carried out by, for example, the method for measuring peroxidase activity as described above.

In the method for the immunological determination of the present invention, the aforementioned buffer, metal ion, salt, saccharide, surfactant, antiseptic agent, protein, protein stabilizing agent and the like can be allowed to coexist in the reaction.

20. Reagent for Determination

The reagent used in the immunological measurement method for determining the analyte of the present invention comprises an enzyme-labeled antibody in which an enzyme is bound as a label to a first antibody that specifically binds to the analyte, said enzyme-labeled antibody substantially containing only one or plural number of enzyme-labeled antibodies selected from the group consisting of enzyme-labeled antibodies having a binding ratio between the first antibody and the enzyme of 1:1, 1:2, 1:3 and 2:1, respectively, and further optionally comprises an antibody in which a separation means is bound to a second antibody that specifically binds to the antigenic determination site. that is different from the antigenic determination site of the analyte to which the first antibody is bound, and/or a reagent for measuring the enzyme activity.

As the enzyme-labeled antibody, enzyme-labeled antibodies substantially containing only one or plural number of enzyme-labeled antibodies selected from the group consisting of enzyme-labeled antibodies having a binding ratio between the first antibody and the enzyme of 1:1, 1:2 and 1:3, respectively are preferred, and enzyme-labeled antibodies substantially containing only one or plural number of enzyme-labeled antibodies selected from the group consisting of enzyme-labeled antibodies having a binding ratio between the first antibody and the enzyme of 1:1 and 1:2, respectively are more preferred.

As the enzyme-labeled antibody, the enzyme-labeled antibody having a binding ratio between the first antibody and the enzyme of 1:1 or 1:2 is particularly preferable. When the enzyme-labeled antibody is a mixture, proportion of the number of molecules of the enzyme-labeled antibody having a binding ratio between the first antibody and the enzyme of 1:1 or 1:2 to the number of molecules of the entire enzyme-labeled antibodies is preferably 50% or greater, more preferably 70% or greater, particularly preferably 90% or greater.

Specific embodiments of the reagent for the measurement of the present invention will be described below.

· Reagent 1
Reagent comprising the solid phased antibody, the enzyme-labeled antibody, and the reagent for measuring activity of the labeled enzyme.
· Reagent 2
Reagent comprising the solid phased antibody, the enzyme-labeled antibody, the IgG polymer, and the reagent for measuring activity of the labeled enzyme.
· Reagent 3
Reagent comprising the solid phased antibody, the enzyme-labeled antibody, mouse IgG, and the reagent for measuring activity of the labeled enzyme.
· Reagent 4
Reagent comprising the solid phased antibody, the enzyme-labeled antibody, the IgG polymer, mouse IgG, and the reagent for measuring activity of the labeled enzyme.

· Reagent 5
Reagent comprising the solid phased antibody, the enzyme-labeled antibody, an inactivated enzyme which is the same enzyme as the enzyme labeling the antibody, and the reagent for measuring activity of the labeled enzyme.
· Reagent 6
Reagent comprising the solid phased antibody, the enzyme-labeled antibody, mouse IgG, an inactivated enzyme which is the same enzyme as the enzyme labeling the antibody, and the reagent for measuring activity of the labeled enzyme.
· Reagent 7

Reagent comprising the solid phased antibody, the enzyme-labeled antibody, the IgG polymer, an inactivated enzyme which is the same enzyme as the enzyme labeling the antibody, and the reagent for measuring activity of the labeled enzyme.

· Reagent 8

Reagent comprising the solid phased antibody, the enzyme-labeled antibody, mouse IgG, the IgG polymer, an inactivated enzyme which is the same enzyme as the enzyme labeling the antibody, and the reagent for measuring activity of the labeled enzyme.

· Reagent 9

Reagent comprising the solid phased antibody, the enzyme-labeled antibody, the reagent for measuring activity of the labeled enzyme, and an authentic sample for the analyte.

· Reagent 10

Reagent comprising the solid phased antibody, the enzyme-labeled antibody, the IgG polymer, the reagent for measuring activity of the labeled enzyme, and the authentic sample for the analyte.

· Reagent 11

Reagent comprising the solid phased antibody, the enzyme-labeled antibody, mouse IgG, the reagent for measuring activity of the labeled enzyme, and the authentic sample for the analyte.

· Reagent 12

Reagent comprising the solid phased antibody, the enzyme-labeled antibody, the IgG polymer; mouse IgG, the reagent for measuring activity of the labeled enzyme, and the authentic sample for the analyte.

· Reagent 13

Reagent comprising the solid phased antibody, the enzyme-labeled antibody, an inactivated enzyme which is the same enzyme as the enzyme labeling the antibody, the reagent for measuring activity of the labeled enzyme, and the authentic sample for the analyte.

· Reagent 14

Reagent comprising the solid phased antibody, the enzyme-labeled antibody, mouse IgG, an inactivated enzyme which is the same enzyme as the enzyme labeling the antibody, the reagent for measuring activity of the labeled enzyme, and the authentic sample for the analyte.

· Reagent 15

Reagent comprising the solid phased antibody, the enzyme-labeled antibody, the IgG polymer, an inactivated enzyme which is the same enzyme as the enzyme labeling the antibody, the reagent for measuring activity of the labeled enzyme, and the authentic sample for the analyte.

· Reagent 16

Reagent comprising the solid phased antibody, the enzyme-labeled antibody, mouse IgG, the IgG polymer, an inactivated enzyme which is the same enzyme as the enzyme labeling the antibody, the reagent for measuring activity of the labeled enzyme, and the authentic sample for the analyte.

Examples of the reagent for measuring activity of the labeled enzyme in the reagent for the measurement of the present invention include e.g., reagents comprising the substrate for the enzyme. Examples of the enzyme in the reagent for measuring the enzyme activity include e.g., the enzymes as described above. Examples of the substrate include e.g., the substrates as described above.

Examples of the authentic sample for the analyte in the reagent for the measurement of the present invention include e.g., aqueous solutions of the analyte adjusted to have a known concentration.

The reagent for immunologically determining an analyte in the present invention may be stored and transported in the form of a kit, and as needed, may contain the aforementioned buffer, a metal ion, a salt, saccharide, a surfactant, an antiseptic agent, protein, a protein stabilizing agent and the like.

Hereinafter, Examples of the present invention will be demonstrated, but the present invention is not limited thereto. In the Examples, the reagent, the enzyme, the serum and the tool provided by the manufacturer as described below were used.

Hybridoma AM92.3: manufactured by PIERCE Inc.
Hybridoma 7G7/B6: manufactured by PIERCE Inc.
α-MEM (Minimum Essential Medium alpha Medium): manufactured by Invitrogen Corporation

96-Well microtiter plate: manufactured by Nalge Nunc International

BSA: manufactured by InterGen

Saccharose: manufactured by KANTO CHEMICAL CO., INC.

Tween 20: manufactured by KANTO CHEMICAL CO., INC.

Gentamicin: manufactured by Wako Pure Chemical Industries, Ltd.

Sodium chloride: manufactured by Wako Pure Chemical Industries, Ltd.

Bioace: manufactured by KUMIAI CHEMICAL INDUSTRY CO., LTD.

Orthophenylenediamine (OPD): manufactured by Sigma-Aldrich, Inc.

Urea peroxide: manufactured by Sigma-Aldrich, Inc.

Proclin: manufactured by Sigma-Aldrich, Inc.

POD: manufactured by Toyobo Co., Ltd.; manufactured by Roche Diagnostics

Sulfuric acid: manufactured by KANTO CHEMICAL CO., INC.

Pepsin: manufactured by Roche Diagnostics

Iminothiolane: manufactured by PIERCE Inc.

EMCS: manufactured by PIERCE Inc.

FBS: manufactured by HyClone

NP-40: manufactured by Calbiochem

Mouse IgG: manufactured by Scantibodies Laboratory, Inc.

Proxel GXL: manufactured by Avecia Ltd.

POD stabilizing buffer: manufactured by DakoCytomation

MES: manufactured by DOJINDO Laboratories

MAK33-IgG1/IgG1 Poly: manufactured by Roche Diagnostics

MAK33-IgG(2b)/Fab(2a) Poly: manufactured by Roche Diagnostics

Normal human serum: manufactured by Aries K.K.

HAMA serum type I: manufactured by Roche Diagnostics

HAMA serum type II: manufactured by Roche Diagnostics

Inactive Poly-POD: manufactured by Roche Diagnostics

Reference Example 1: Preparation and Purification of Anti-sIL-2R Monoclonal Antibody

The hybridoma AM92.3 and the hybridoma 7G7/B6 which produce an anti-sIL-2R , monoclonal antibody were transplanted into peritoneal cavity of mouse treated with pristane or the like, respectively, and the ascitic fluid was recovered. A monoclonal antibody was purified from the ascitic fluid using a protein A column: rProtein A Sepharose Fast Flow (manufactured by Amersham Bioscience K.K.) according to a common procedure. The monoclonal antibody obtained from the hybridoma AM92.3 was designated as KTM-302 antibody, and the monoclonal antibody obtained from the hybridoma 7G7/B6 was designated as KTM-303 antibody.

Reference Example 2: Preparation of sIL-2R

Cryopreserved lymphoma cell strain U937 (manufactured by Dai Nippon Pharmaceutical Co., Ltd.) which is known to express and secrete sIL-2R was quickly thawed in a 37°C water bath, and transferred to a 15 mL sterile tube. Thereto was added 10 mL of $\alpha$-MEM containing 10% FBS, penicillin and streptomycin, and the mixture was gently allowed to suspend. After a centrifugal separation (1200 rpm) at a room temperature for 5 minutes, the supernatant was eliminated by aspiration. To the residue was added 10 mL of the same medium to permit suspension. Thus resulting all amount of the cell suspension liquid was transferred to a 25 cm$^2$ T flask, and cultured in a carbon dioxide gas culture apparatus (5% $CO_2$, 37°C) for 2 to 3 days. Thereafter, expansion culture was sequentially carried out into a 150 cm$^2$ T flask, and then a 225 cm$^2$ T flask. After ascertaining 100% confluence of the cells in the 225 cm$^2$ T flask, the culture supernatant was recovered into a sterile vessel, and subjected to centrifugal separation (1200 rpm) at 4°C for 10 minutes. The supernatant obtained by the centrifugal separation was transferred to a sterile vessel, and stirred gently for 15 minutes. Thereafter, the supernatant was subjected to a filtration treatment with a 0.2 $\mu$m filter to provide an sIL-2R standard substance.

In accordance with a document [J. Immunol., 135, 3172-3177 (1985)], value for the standard substance was determined on the basis of the amount, assumed to be 1000 U/mL, of sIL-2R included in the cell free culture supernatant (without dilution) of normal human IL-2 dependent T cells stimulated with 10% IL-2 for 4 days.

Reference Example 3: Preparation of Solid Phase with Immobilized Anti-SiL-2R Monoclonal Antibody

The KTM-302 antibody was diluted with PBS so that the final concentration became 4 $\mu$g/mL, and each 100

μL of the diluted solution was added to each well of a 96-well microtiter plate for use as the solid phase. After standing still at room temperature overnight, washing with a blocking solution (10 mmol/L phosphate buffer, pH 7.2, containing 1% BSA, 5% saccharose, 0.05% Tween 20, 0.01% gentamicin sulfate) was carried out, followed by adding 200 μL of the blocking solution, whereby the blocking at room temperature was carried out by still standing overnight. After removing the blocking solution, an anti-sIL-2R monoclonal antibody-immobilized solid phase (plate) was prepared through drying by a vacuum dryer for 3 days.

Example 1

(1) Preparation of POD-labeled Anti-sIL-2R Monoclonal F(ab')$_2$ Antibody

The KTM-303 antibody prepared in Reference Example 1 was digested with 0.01% pepsin, and thereafter F(ab')$_2$ was separated and purified on an HPLC system (manufactured by Hitachi, Ltd.) using a G3000SW column (manufactured by Tosoh Corporation; φ 21.5 mm x 60 cm). Thus resulting F(ab')$_2$ in an amount of 4 mg was dialyzed against 100 mmol/L borate buffer (pH 8.0).
The anti-sIL-2R monoclonal F(ab')$_2$ antibody and POD (manufactured by Toyobo Co., Ltd.) were bound according to a maleimide method as described below.
The F(ab')$_2$ was sulfhydrylated using iminothiolane, and unreacted iminothiolane was removed by a Sephadex G-25 column (manufactured by Amersham Bioscience K.K.). POD was maleimidated using a maleimide reagent EMCS, and unreacted EMCS was removed by a Sephadex G-25 column. The aforementioned sulfhydrylated F(ab')$_2$ monoclonal antibody and the maleimidated POD were mixed, and allowed to react at 30°C for 30 minutes to prepare a POD-labeled anti-sIL-2R monoclonal F(ab')$_2$ antibody.

(2) Separation of POD-labeled Anti-SiL-2R Monoclonal F(ab')$_2$ Antibody by Gel Filtration Column Chromatography

The molecular weight of the F(ab')$_2$ antibody is about 92 kDa, and the molecular weight of POD is about 44 kDa. Therefore, it is calculated that the molecular weight of the enzyme-labeled antibody having a binding ratio between the antibody and POD of 1:1 is about 136 kDa; that of 1:2 is about 180 kDa; that of 1:3 is about 224 kDa; that of 1:4 is about 268 kDa; that of 1:5 is about 312 kDa; that of 2:1 is about 228 kDa; that of 2:2 is about 272 kDa; that of 2:3 is about 316 kDa; and that of 3:1 is about 320 kDa.
The enzyme-labeled antibody prepared with the method described in above (1) was fractionated by an HPLC system (manufactured by Hitachi, Ltd.) having a G3000SW column (manufactured by Tosoh Corporation; φ 21.5 mm x 60 cm). HPLC was performed using a 0.1 mol/L phosphate buffer (pH 7.4) as a mobile phase at a flow rate of 3 mL/minutes, at room temperature. Recovery of the fraction was carried out to obtain 3 mL/fraction. Employed molecular weight markers were a kit for calibration on high molecular weight gel filtration (HMW Gel Filtration Calibration Kit, manufactured by Amersham Bioscience K.K.) and a kit for calibration on low molecular weight gel filtration (LMW Gel Filtration Calibration Kit, manufactured by Amersham Bioscience K.K.).
Measured absorbances at 280 nm of each of the fractions are illustrated in Fig. 1. As shown in Fig. 1, absorbance peaks were found in 1) fraction 33, 2) fraction 40, 3) fraction 43, 4) fraction 48, and 5) fraction 52.
The molecular weight of each of the fractions showing the aforementioned absorbance peak, as deduced by comparison with the molecular weight marker was 1) 250 kDa or greater, 2) about 170 kDa, 3) about 140 kDa, 4) about 100 kDa, and 5) about 40 kDa, respectively. Therefore, it was estimated that: 1) the enzyme-labeled antibody in the fraction 33 is the labeled antibody in which F(ab')$_2$ and POD are polymerized; 2) the enzyme-labeled antibody in the fraction 40 is the labeled antibody in which 2 molecules of POD are bound to 1 molecule of F(ab')$_2$; 3) the enzyme-labeled antibody in the fraction 43 is the enzyme-labeled antibody in which 1 molecule of POD is bound to 1 molecule of F(ab')$_2$; 4) the fraction 48 contains unreacted F(ab')$_2$; and 5) fraction 52 contains unreacted POD.

(3) Identification of Enzyme-labeled Antibody in Each Fraction by SDS-PAGE

For each of the fractions of 32 to 52 obtained in (2) as described above, a sample for electrophoresis was prepared so that the amount of the protein derived from each fraction became 2 to 3 μg/lane. To this sample was added a sample buffer for SDS-PAGE, [1 mol/L Tris buffer, pH 6.8, containing 8% SDS (manufactured by Wako Pure Chemical Industries, Ltd.), 24% 2-mercaptoethanol (manufactured by Nacalai Tesque, Inc.), and 40% glycerol (manufactured by KANTO CHEMICAL CO., INC.)] in a quarter amount, and heated at 95°C for 5 minutes. The sample subjected to the heat treatment was applied on a gel for SDS-PAGE [PAGEL SPG-520L (manufactured by ATTO Corporation)] in a volume of 20 μL/lane, and the electrophoresis was conducted at 20 mA per the gel. Bands were detected according to a common procedure. The molecular weight marker employed was "Prestained Broad Range" (manufactured by Bio-Rad Laboratories, Inc.,: 250 kDa, 150 kDa, 100 kDa, 75 kDa, 50 kDa, 37 kDa, 25 kDa, 16 kDa, 10 kDa).

From the band position appeared on SDS-PAGE, using the molecular weight marker, it was identified that: the enzyme-labeled antibody in the fractions 32 to 35 is a mixture of the enzyme-labeled antibodies that are high polymers having a binding ratio between $F(ab')_2$ and POD of 1:5, 2:3, 3:1 and the like, respectively (the bands were observed in a smear pattern); the enzyme-labeled antibody in the fraction 36 is the enzyme-labeled antibodies having a binding ratio between $F(ab')_2$ and POD of 1:4 and 2:2, respectively; the enzyme-labeled antibody in the fraction 37 is a mixture of the enzyme-labeled antibodies having a binding ratio between $F(ab')_2$ and POD of 1:4, 1:3, 2:1 and 2:2, respectively.

Similarly, it was identified that: the enzyme-labeled antibody in the fraction 38 is a mixture of the enzyme-labeled antibodies having a binding ratio between $F(ab')_2$ and POD of 1:3 and 2:1, respectively; the enzyme-labeled antibody in the fraction 39 is a mixture of the enzyme-labeled antibodies having a binding ratio between $F(ab')_2$ and POD of 1:2, 1:3 and 2:1, respectively; the enzyme-labeled antibody in the fractions 40 to 41 is the enzyme-labeled antibody having a binding ratio between $F(ab')_2$ and POD of 1:2; the enzyme-labeled antibody in the fraction 42 is a mixture of the enzyme-labeled antibodies having a binding ratio between $F(ab')_2$ and POD of 1:2 and 1:1, respectively; the enzyme-labeled antibody in the fractions 43 to 46 is the enzyme-labeled antibody having a binding ratio between $F(ab')_2$ and POD of 1:1.

Moreover, it was identified that: the protein in the fraction 47 is a mixture of the enzyme-labeled antibody having a binding ratio between $F(ab')_2$ and POD of 1:1, and unreacted $F(ab')_2$; the protein in the fractions 48 to 51 is unreacted $F(ab')_2$; and the protein in the fraction 52 is unreacted POD.

(4) Preparation of Calibration Curve for Determining sIL-2R Using Enzyme-labeled Antibody in Each Fraction

To each well of the solid phased plate prepared in Reference Example 3 was added each 50 $\mu$L of a standard substance solution (10 mmol/L phosphate buffer, pH 7.5, containing 150 mmol/L sodium chloride, 4% BSA, 1% saccharose and 0.01% Bioace) including 0 U/mL, 200 U/mL, 400 U/mL, 1600 U/mL, 3200 U/mL or 6400 U/mL sIL-2R. Each 50 $\mu$L of the labeled antibody solution (24 to 140 ng/mL) prepared by diluting the labeled antibody in each fraction of (2) as described above using an enzyme-labeled antibody diluent [100 mmol/L acetate buffer, pH 6.0, containing 150 mmol/L sodium chloride, 10% FBS, 0.2% NP-40, 100 $\mu$g/mL mouse IgG, 0.2% Proxel GXL, 10% POD stabilizing buffer and 0.16 $\mu$g/mL POD (manufactured by Roche Diagnostics)] was added to each well, followed by shaking using a horizontal rotary shaker at room temperature for 90 minutes (140 to 160 rpm). After washing each well with a wash liquid (10 mmol/L phosphate buffer, pH 6.8, containing 150 mmol/L sodium chloride, 0.05% Tween 20), 100 $\mu$L of an OPD solution (100 mmol/L phosphate-citrate buffer, pH 4.4, containing 2 mg/mL OPD, 0.75 g/L urea peroxide, 0.01% Proclin 300) was added to each well, and the reaction was allowed by standing still at room temperature for 30 minutes. As a reaction stopping liquid, 50 $\mu$L of 1 mol/L sulfuric acid was added, and absorbance of the reaction liquid was measured at a main wavelength of 490 nm, and a sub wavelength of 660 nm. A calibration curve was prepared produced from the antigen concentration and the absorbance for every labeled antibody in each fraction.

(5) Measuring sIL-2R in Sample Using POD-labeled Anti-sIL-2R Monoclonal Antibody

Using 50 $\mu$L of HAMA serum type I (Lot. 92069721), HAMA serum type II (Lot. 14482684) and normal human serum (Lot. 101001-2) as a sample, sIL-2R in each serum sample was measured using the labeled antibody in each fraction in (2) described above according to the aforementioned method in (4). sIL-2R measured values (U/mL) are shown in Table 1.

[Table 1]

Table 1

| Fraction number | sIL-2R measured value (U/mL) | | |
| --- | --- | --- | --- |
| | HAMA serum type I | HAMA serum type II | Normal human serum |
| 32 | 16142 | 1005 | 230 |
| 33 | 3688 | 259 | 270 |
| 34 | 1881 | 196 | 266 |
| 35 | 1502 | 176 | 290 |
| 36 | 913 | 158 | 280 |
| 37 | 662 | 144 | 284 |
| 38 | 508 | 144 | 284 |

(continued)

| Fraction number | sIL-2R measured value (U/mL) | | |
| --- | --- | --- | --- |
| | HAMA serum type I | HAMA serum type II | Normal human serum |
| 39 | 435 | 135 | 311 |
| 40 | 367 | 142 | 310 |
| 41 | 326 | 129 | 321 |
| 42 | 272 | 133 | 322 |
| 43 | 287 | 134 | 325 |
| 44 | 301 | 134 | 332 |
| 45 | 310 | 137 | 322 |
| 46 | 286 | 148 | 346 |

In fractions 32 to 35 including the enzyme-labeled antibodies that are high polymers having a binding ratio between the antibody and the enzyme of 1:5, 2:3, 3:1 and the like, respectively, the measured values of sIL-2R in the HAMA serum type I were greater than 1000 U/mL. The fraction 32 expected to include particularly highly polymerized ones exhibited a high value of about 16000 U/mL. In addition, the fraction 36 identified as having the binding ratio between the antibody and the enzyme of 1:4 or 2:2 also exhibited a considerably high value.

However, it was indicated that the enzyme-labeled antibody in the fractions 38 to 46 identified to include only one or plural number of enzyme-labeled antibodies selected from the group consisting of the enzyme-labeled antibodies having a binding ratio between the antibody and the enzyme of 1:3, 2:1, 1:2 and 1:1, respectively suppressed the nonspecific reaction appeared in the HAMA serum type I as the measured values of sIL-2R were almost 500 U/mL or less. In particular, equilibrium state at about 300 U/mL was shown around from the fraction 40 including the enzyme-labeled antibody in which 2 molecules of POD were bound to 1 molecule of $F(ab')_2$ to the fraction 46 including the enzyme-labeled antibody in which 1 molecule of POD was bound to 1 molecule of $F(ab')_2$.

These results suggest that the nonspecific reaction strongly appeared in the HAMA serum type I can be effectively suppressed by using the enzyme-labeled antibody in which a small number of labeling enzyme molecules are bound per molecule of the antibody, preferably, the enzyme-labeled antibody substantially containing only one or plural number of enzyme-labeled antibodies selected from the group consisting of enzyme-labeled antibodies having a binding ratio between the first antibody and the enzyme of 1:1, 1:2, 1:3 and 2:1, respectively, more preferably, the enzyme-labeled antibody substantially containing only one or plural number of enzyme-labeled antibodies selected from the group consisting of enzyme-labeled antibodies having a binding ratio between the first antibody and the enzyme of 1: 1, 1:2 and 1:3, respectively, and particularly preferably, the enzyme-labeled antibody substantially containing only one or plural number of enzyme-labeled antibodies selected from the group consisting of enzyme-labeled antibodies having a binding ratio between the first antibody and the enzyme of 1:1 and 1:2, respectively.

Example 2

Effect of Mouse IgG in HAMA Nonspecific Reaction

Effect of addition of mouse IgG on the suppression of the nonspecific reaction caused by HAMA was studied. The enzyme-labeled antibody in the fractions 40 to 46 prepared in Example 1 was diluted with an enzyme-labeled antibody diluent [75 mmol/L MES buffer (pH 6.5) containing 150 mmol/L sodium chloride, 10% FBS, 0.2% NP-40, mouse IgG, 0.2% Proxel GXL, 10% POD stabilizing buffer, 0.16 μg/mL POD (manufactured by Roche Diagnostics)] to adjust the mouse IgG concentrations of 0, 20, 40, 60, 80, 100, 200 μg/mL, respectively.

Using each 50 μL of the HAMA serum type I (Lot. 92069721), HAMA serum type II (Lot. 14482684) and normal human serum (Lot. 101001-2), the sIL-2R value in each serum was determined in a similar manner to Example 1 (5). Results are shown in Table 2.

[Table 2]

Table 2

| Mouse IgG concentration | sIL-2R measured value (U/mL) | | |
|---|---|---|---|
| | HAMA serum type I | HAMA serum type II | Normal human serum |
| 0 | 452 | 9063 | 351 |
| 20 | 458 | 437 | 333 |
| 40 | 456 | 338 | 336 |
| 60 | 452 | 296 | 327 |
| 80 | 403 | 268 | 320 |
| 100 | 405 | 264 | 334 |
| 200 | 400 | 268 | 345 |

Although the measured value of the HAMA serum type II without addition of mouse IgG was 9000 U/mL or greater suggesting the nonspecific reaction caused by HAMA, addition of 20 μg/mL mouse IgG suppressed the non-specific reaction caused by HAMA to lower the value to 437 U/mL. However, by further adding the mouse IgG, the measured value of the HAMA serum was lowered, and the value of the sIL-2R reached to the equilibrium by the addition of 80μ g/mL or greater. Also in the HAMA serum type I, the measured value reached to the equilibrium at 80 μg/mL, but a great change as the case of the HAMA serum type II was not observed. Moreover, the measured value of the normal human serum was constant independently of the mouse IgG concentration, and thus it was ascertained that the addition of mouse IgG did not affect the measurement system up to 200 μg/mL. From these results, it was revealed that the addition of 80 μg/mL or greater mouse IgG could sufficiently suppress the nonspecific reaction caused by the HAMA serum type II. Also in the case of the HAMA serum type I, suppressive effect was verified.

Example 3

Study on Effect of Highly Polymerized Mouse IgG and Optimum Concentration of Highly Polymerized Mouse IgG in HAMA Nonspecific Reaction

Addition of the highly polymerized mouse IgG affecting the suppression of the nonspecific reaction caused by HAMA was studied. The enzyme-labeled antibody in the fractions 40 to 46 prepared in Example 1 was diluted with an enzyme-labeled antibody diluent [75 mmol/L MES buffer, pH 6.5, containing 150 mmol/L sodium chloride, 10% FBS, 0.2% NP-40, 100 μg/mL mouse IgG, highly polymerized mouse IgG, 0.2% Proxel GXL, 10% POD stabilizing buffer and 0.16 μg/mL POD (manufactured by Roche Diagnostics)]. When MAK33-IgG1/IgG1 Poly was used as the polymerized mouse IgG, the enzyme-labeled antibody solution was prepared so that the concentration thereof became 0, 75, 100, 125, 150, 175, 200, 300 μg/mL, respectively. When MAK33-IgG(2b)/Fab(2a) Poly was used as the polymerized mouse IgG, the enzyme-labeled antibody was prepared so that the concentration thereof became 0, 5, 50, 100 μg/mL, respectively.
Using each 50 μL of the HAMA serum type I (Lot. 90643629), HAMA serum type II (Lot. 92069831) and normal human serum (Lot. 101001-2), sIL-2R in each serum was determined in a similar manner to Example 1 (5). Effects of addition of MAK33-IgG1/IgG1 Poly are shown in Table 3.
[Table 3]

Table 3

| MAK33-IgG1/IgG1 Poly (μg/mL) | sIL-2R measured value (U/mL) | | |
|---|---|---|---|
| | HAMA I | HAMA II | Normal human serum |
| 0 | 435 | 281 | 340 |
| 75 | 285 | 270 | 339 |
| 100 | 268 | 257 | 317 |
| 125 | 248 | 247 | 331 |
| 150 | 241 | 247 | 329 |

(continued)

| MAK33-IgG1/IgG1 Poly (μg/mL) | sIL-2R measured value (U/mL) | | |
|---|---|---|---|
| | HAMA I | HAMA II | Normal human serum |
| 175 | 237 | 247 | 329 |
| 200 | 227 | 229 | 324 |
| 300 | 217 | 224 | 322 |

Consequently, as shown in Table 3, the measured value of sIL-2R in the HAMA serum type I without addition of MAK33-IgG1/IgG1 Poly was 435 U/mL, but the addition of MAK33-IgG1/IgG1 Poly lowered the measured value in a concentration dependent manner. The measured value of sIL-2R became almost constant at the additive concentration of 125 μg/mL or greater.

Furthermore, the measured value on the normal human serum did not vary even though MAK33-IgG1/IgG1 Poly was added to 300 μg/mL. Therefore, it was verified that the addition of MAK33-IgG1/IgG1 Poly up to 300 μg/mL did not affect the measured value.

These results revealed that the addition of 125 μg/mL or more MAK33-IgG1/IgG1 Poly to the enzyme-labeled antibody solution could sufficiently suppress the nonspecific reaction caused by the HAMA serum type I.

Further, effects of the addition of MAK33-IgG(2b)/Fab(2a) Poly are shown in Table 4.

[Table 4]

Table 4

| MAK33-IgG(2b)/Fab(2a) Poly (μg/mL) | sIL-2R measured value (U/mL) | | |
|---|---|---|---|
| | HAMA I | HAMA II | Normal human serum |
| 0 | 556 | 176 | 343 |
| 5 | 516 | 175 | 355 |
| 50 | 348 | 175 | 356 |
| 100 | 235 | 171 | 345 |

As shown in Table 4, also in the case in which MAK33-IgG(2b)/Fab(2a) Poly was used in place of MAK33-IgG1/IgG1 Poly, the measured value of sIL-2R in the HAMA serum type I was similarly lowered.

Accordingly, it was revealed that addition of not only IgG1 but also other subtype IgG polymer could suppress the nonspecific reaction caused by the HAMA serum type I.

Example 4

Measuring sIL-2R in HAMA Serum Specimen (1)

Using the enzyme-labeled antibody solution prepared by diluting the enzyme-labeled antibody in the fractions 40 to 46 prepared in Example 1 with a labeled antibody diluent (75 mmol/L MES buffer, pH 6.5, containing 150 mmol/L sodium chloride, 10% FBS, 0.2% NP-40, 100 μg/mL mouse IgG, 200 μg/mL MAK33-IgG1/IgG1 Poly, 0.2% Proxel GXL, 10% POD stabilizing buffer and 0.16 μg/mL POD), sIL-2R in the HAMA serum type I (Lot. 90643637) and in the HAMA serum type II (Lot. 92069840) were determined in a similar manner to Example 1 (5). Hereinafter, the enzyme-labeled antibody solution prepared as described above, and the reagent other than the enzyme-labeled antibody solution used in the method described in Example 1 (4) (the solid phased plate prepared in Reference Example 3, the standard substance solution in Example 1 (4), washing solution, OPD solution and reaction stopping solution) are collectively referred to as "reagent of Example 4". Compositions of the reagent of Example 4 are described below.

Reagent of Example 4

Anti-sIL-2R Antibody-immobilized Plate: 96-well microtiter plate of 8 well x 12 strips, solid-phased with 100 μL/well of 4 μg/mL KTM-302 antibody

Standard Substance Solution: standard substance solution (10 mmol/L phosphate buffer, pH 7.5, containing 150 mmol/L sodium chloride, 4% BSA, 1% saccharose and 0.01% Bioace) containing 0 U/mL, 200 U/mL, 400 U/mL, 1600. U/mL, 3200 U/mL, 6400 U/mL sIL-2R, respectively

Enzyme-labeled Antibody Solution: labeled antibody diluent (75 mmol/L MES buffer, pH 6.5, containing 150 mmol/L sodium chloride, 10% FBS, 0.2% NP-40, 100 $\mu$g/mL mouse IgG, 200 $\mu$g/mL MAK33-IgG1/IgG1 Poly, 0.2% Proxel GXL, 10% POD stabilizing buffer and 0.16 $\mu$g/mL POD) including a predetermined amount (24 to 140 ng/mL) of the POD-labeled antibody in the fractions 40 to 46 in Example 1

Washing solution: 10 mmol/L phosphate buffer, pH 6.8, containing 150 mmol/L sodium chloride, 0.05% Tween 20

OPD Solution: 100 mmol/L phosphate-citrate buffer, pH 4.4, containing 2 mg/mL OPD, 0.75 g/L urea peroxide, 0.01% Proclin 300

Reaction Stopping solution: 1 mol/L sulfuric acid

Also, measuring sIL-2R in each serum using Immulyze IL-2R (manufactured by DPC Inc.,) was separately carried out. Results are shown in Table 5 .

[Table 5]

Table 5

| Reagent | sIL-2R measured value (U/mL) | |
| --- | --- | --- |
| | HAMA serum type I | HAMA serum type II |
| Immulyze IL-2R | 408 | 906 |
| Reagent of Example 4 | 200 | 219 |

Consequently, sIL-2R measured value using Reagent of Example 4 in the HAMA serum was lower than the measured value using the Immulyze IL-2R kit, and it was suggested that the nonspecific reaction caused by HAMA could be sufficiently suppressed.

Example 5

Measuring sIL-2R in Specimen including HAMA (2)

Measuring sIL-2R in specimens to which the HAMA serum was added in a varying amount to the normal human serum was carried out, and effects of the HAMA concentration on the measurement value were determined.

First, the HAMA serum type I (Lot. 90643656), the HAMA serum type II (Lot. 92069858) and the normal human serum (F41489B) for use in preparing the specimen were provided as samples, and sIL-2R in each sample was measured using the reagent of Example 4, while HAMA was measured using ImmuSTRIP HAMA Fragment (manufactured by Immunomedics, Inc.) that is a sandwich ELISA kit for HAMA measurement. Respective measured results, and theoretical values of sIL-2R and HAMA concentration in 5 x HAMA serum (theoretical value = measured value x 5) are shown in Table 6 because 5-fold concentrated HAMA serum (hereinafter, referred to as 5 x HAMA serum) was used for the preparation of the specimen.

[Table 6]

Table 6

| Sample | sIL-2R (U/mL) | | HAMA ($\mu$g/mL) | |
| --- | --- | --- | --- | --- |
| | Measured value | Theoretical value for 5 x HAMA serum | Measured value | Theoretical value for 5 x HAMA serum |
| HAMA serum type I | 260.5 | 1302.7 | 26.1 | 130.5 |
| HAMA serum type II | 275.5 | 1377.7 | 12.5 | 62.5 |
| Normal human serum | 266.0 | - | 0.0 | - |

Using a mixture of the 5 x HAMA serum (type I or type II) and the normal human serum at a ratio of 1:1, 1:2 and 1:4, respectively, as a sample, sIL-2R in each sample was measured using the reagent of Example 4 and Immulyze IL-2R. On the other hand, theoretical value of sIL-2R in each sample was determined which was derived from the sIL-2R concentration in the 5 x HAMA serum and the normal human serum as determined above and from the mixing ratio with the sample serum. Further, percentage influence (%) on the measured value was determined based on the ratio of the measured value to the theoretical value of sIL-2R in each sample. The concentration of HAMA in each sample was also determined by calculation similarly to the theoretical value of sIL-2R.

Theoretical Value of sIL-2R When the Mixing Ratio of 5 x HAMA Serum to Normal Human Serum is 1:a

= [sIL-2R concentration in 5 x HAMA serum (theoretical

value) + (sIL-2R concentration in normal human serum) x

a)]/ (1 + a)

Percentage Influence (%)

= [(measurement value of sIL-2R concentration/

theoretical value of sIL-2R concentration) x 100] - 100

The HAMA concentration in each sample, the theoretical value and measured value of the sIL-2R concentration, and the percentage influence are shown for each case in which the reagent of Example 4 was used (in Table 7), and in which Immulyze IL-2R was used (in Table 8).

[Table 7]

Table 7 Measurement with Reagent of Example 4

| Sample | | | sIL-2R | | |
|---|---|---|---|---|---|
| HAMA serum | Mixing ratio of serum 5 x HAMA: normal human | HAMA. (μg/mL) | Theoretical value (U/mL) | Measured value (U/mL) | Percentage influence on measured value |
| HAMA serum type | 1:4 | 26.1 | 473.3 | 514.1 | 8.6% |
| | 1:2 | 43.5 | 611.6 | 660.4 | 8.0% |
| | 1:1 | 65.3 | 784.3 | 837.0 | 6.7% |
| HAMA serum type II | 1:4 | 12.5 | 488.3 | 511.5 | 4.7% |
| | 1:2 | 20.8 | 636.6 | 663.0 | 4.2% |
| | 1:1 | 31.1 | 821.9 | 847.3 | 3.1% |

Table 8 Measurement with Immulyze IL-2R

| Sample | | | sIL-2R | | |
|---|---|---|---|---|---|
| HAMA serum | Mixing ratio of serum 5 x HAMA: normal human | HAMA (μg/mL) | Theoretical value (U/mL) | Measured value (U/mL) | Percentage influence on measured value |
| HAMA serum type I | 1:4 | 26.1 | 504.5 | 709.0 | 40.5% |
| | 1:2 | 43.5 | 637.6 | 890.0 | 39.6% |
| | 1:1 | 65.3 | 803.8 | 1040.0 | 29.4% |
| HAMA serum type | 1:4 | 12.5 | 519.5 | 1150.0 | 121.3% |
| | 1:2 | 20.8 | 662.6 | 1630.0 | 146.0% |
| | 1:1 | 31.1 | 841.4 | 2200.0 | 161.5% |

In connection with the HAMA serum type I, it was revealed that the percentage influence on the measured value was 10% or less even though the HAMA concentration was elevated to 65.3 μg/mL (2.5 times HAMA included in commercially available HAMA serum type I) when the measurement was carried out with the reagent of Example 4, however, when the measurement was carried out with Immulyze IL-2R, percentage influence on the measurement value was 30 to 40%, suggesting the influence of HAMA. Moreover, in connection with the HAMA serum type II, it was revealed that the percentage influence on the measurement value was 5% or less even though the HAMA concentration was elevated to 31.1 $\mu$g/mL (2.5 times HAMA included in commercially available HAMA serum type II) when the measurement was carried out with the reagent of Example 4, however, when the measurement was carried out with Immulyze IL-2R, percentage influence on the measurement value was 120 to 160%, suggesting great influence of HAMA. These results also indicated that the suppression of the nonspecific reaction caused by HAMA is insufficient with Immulyze IL-2R, but the reagent of Example 4 could sufficiently suppress the nonspecific reaction caused by HAMA.

Example 6

sIL-2R Measurement Kit

Reagent kit comprising the following each reagent was prepared.

1) Anti-sIL-2R Antibody-Immobilized Plate

96-Well microtiter plate of 8 well x 12 strips, solid-phased with 100 $\mu$L/well of 4 $\mu$g/mL KTM-302 antibody

2) Enzyme-labeled Antibody

Composition: labeled antibody diluent (75 mmol/L MES buffer, pH 6.5, containing 150 mmol/L sodium chloride, 10% FBS, 0.2% NP-40, 100 $\mu$g/mL mouse IgG, 200 $\mu$g/mL MAK33-IgG1/IgG1 Poly, 0.2% Proxel GXL, 10% POD stabilizing buffer and 0.16 $\mu$g/mL POD) including the POD-labeled antibody in the fractions 40 to 46 in Example 1 in a predetermined amount (24 to 140 ng/mL)
Volume: 6 mL/bottle

3) Standard Substance

Freeze-dried product of standard substance solution (10 mmol/L phosphate buffer, pH 7.5, containing 150 mmol/L sodium chloride, 4% BSA, 1% saccharose and 0.01% Bioace) corresponding to 0.5 mL/vial each containing 0 U/mL, 200 U/mL, 400 U/mL, 1600 U/mL, 3200 U/mL, 6400 U/mL sIL-2R

4) Specimen Diluent

Composition: 10 mmol/L phosphate buffer (pH 7.45) containing 150 mmol/L sodium chloride, 4% BSA, 1% saccharose, 0.2% Proxel GXL and 20 $\mu$g/mL mouse IgG
Volume: 6 mL/bottle.

5) Color Development Substrate

OPD tablet (manufactured by Sigma-Aldrich, Inc.), 10 mg/tablet x 6

6) Color Development Substrate Dissolution Liquid

Composition: 100 mmol/L phosphate-citrate buffer (pH 4.4) containing 0.75 g/L urea peroxide and 0.1% Proclin 300
Volume: 30 mL/bottle

7) Reaction Stopping Liquid

Composition: 1 mol/L sulfuric acid
Volume: 6 mL/bottle

8) Washing solution

10 mmol/L Phosphate buffer (pH 6.8) containing 150 mmol/L sodium chloride and 0.05% Tween 20

Example 7

Suppression of Nonspecific Reaction Resulting from Antibody which Reacts with POD

(1) Specimen which Exhibits Nonspecific Reaction Caused by Factor other than HAMA

Specimen A which is a human serum was diluted with a specimen diluent (10 mmol/L phosphate buffer, pH 7.45, containing 50 mmol/L sodium chloride, 4% BSA, 1% saccharose, 0.2% Proxel GXL and 20 $\mu$g/mL mouse IgG) to 1/1, 1/4, 1/8. Then, each sIL-2R concentration was measured with the reagent of Example 4. Table 9 shows the measurement values, and calculated values of sIL-2R in the specimen neat liquid determined from the measurement value and the dilution ratio. sIL-2R calculated values on the specimen neat liquid varied depending on the dilution ratio showing inferior linearity with respect to the dilution, therefore, it was revealed that a nonspecific reaction was caused.

[Table 9]

Table 9

| Dilution ratio | sIL-2R measured value (U/ML) | sIL-2R calculated value in specimen neat liquid (U/mL) |
|---|---|---|
| 1/1 | 11794 | 11794 |
| 1/4 | 2169 | 8676 |
| 1/8 | 486 | 3888 |

In order to verify whether or not the nonspecific reaction in the specimen A results from HAMA, HAMA concentration included in the specimen A was measured using HAMA ELISA (manufactured by Roche Diagnostics) that is a HAMA measurement kit. Results are shown in Table 10. With respect to the HAMA serum type I and the HAMA serum type II, high concentration of HAMA was detected, however, the HAMA value in the specimen A was below 5 ng/mL that is a detection limit, ruling out the presence of HAMA. Therefore, the nonspecific reaction in the specimen A was believed to result from a factor other than HAMA.

[Table 10]

Table 10

| Sample | HAMA measured value (ng/mL) |
|---|---|
| Specimen A | 3.5 |
| HAMA serum type I | 3603 |
| HAMA serum type II | 18033 |

(2) Suppression of Nonspecific Reaction in Specimen A

The specimen A was diluted with the specimen diluent to 1/2, 1/4, 1/6, 1/11, 1/21, 1/31, and sIL-2R was measured using a sIL-2R measurement reagent having the following constitution (hereinafter, referred to as sIL-2R measurement reagent B) in which POD-labeled antibody which was labeled according to a periodic acid method was used. Thus, the calculated values of sIL-2R in the specimen neat liquid were determined from thus resulting measurement value and the dilution ratio. The sIL-2R measurement reagent B had the same constitution as that of the reagent of Example 4 except for the enzyme-labeled antibody solution, and the POD-labeled antibody therein was obtained without subjecting the antibody to separation by gel filtration.

sIL-2R Measurement Reagent B

Anti-sIL-2R Antibody-immobilized Plate: 96-well microtiter plate of 8 well x 12 strips, solid-phased with 100 $\mu$L/well of 4 $\mu$g/mL KTM-302 antibody
Standard Substance Solution: standard substance solution (10 mmol/L phosphate buffer, pH 7.5, containing 150 mmol/L sodium chloride, 4% BSA, 1% saccharose and 0.01% Bioace) containing 0 U/mL, 200 U/mL, 400 U/mL, 1600 U/mL, 3200 U/mL, 6400 U/mL sIL-2R, respectively
Enzyme-labeled Antibody Solution: labeled antibody diluent (10 mmol/L phosphate buffer, pH 7.5, containing 150 mmol/L sodium chloride, 10% FBS, 0.2% NP-40, 20 $\mu$g/mL mouse IgG, 0.2% Proxel GXL, 10% POD stabilizing buffer and 0.16 µg/mL POD) including a predetermined amount (24 to 140 ng/mL) of the POD-labeled antibody which was labeled according to the periodic acid method

Wash Liquid: 10 mmol/L phosphate buffer, pH 6.8, containing 150 mmol/L sodium chloride, 0.05% Tween 20

OPD Solution: 100 mmol/L phosphate-citrate buffer, pH 4.4, containing 2 mg/mL OPD, 0.75 g/L urea peroxide, 0.01% Proclin 300

Reaction Stopping Liquid: 1 mol/L sulfuric acid

On the other hand, as a result of nonspecific reaction absorption test in which the specimen A was diluted with a mouse serum to 1/2, 1/4, 1/8, and the measurement of sIL-2R was carried out using the reagent of Example 4, theoretical value of sIL-2R included in the specimen A was calculated to be 3624 U/mL. With respect to the calculated values of sIL-2R in the specimen neat liquid at each dilution ratio as described above (1) and in the case in which the reagent of Example 4 was used, ratio to the theoretical value of 3624 U/mL was determined. The ratios together with the sIL-2R calculated values and the sIL-2R measurement values on the specimen neat liquid are shown in Table 11 (when the reagent of Example 4 was used) and Table 12 (when the sIL-2R measurement reagent B was used).

[Table 11]

Table 11

| Dilution ratio | Measured value | sIL-2R calculated value in specimen neat liquid (U/mL) | Ratio of calculated value to theoretical value of 3624 U/mL (%) |
|---|---|---|---|
| 1/1 | 11794 | 11794 | 325 |
| 1/4 | 2169 | 8676 | 239 |
| 1/8 | 486 | 3888 | 107 |

[Table 12]

Table 12

| Dilution ratio | Measured value | sIL-2R calculated value in specimen neat liquid(U/mL) | Ratio of calculated value to theoretical value of 3624 U/mL (%) |
|---|---|---|---|
| 1/2 | 10657 | 21314 | 588 |
| 1/4 | 8623 | 34492 | 952 |
| 1/6 | 1233 | 7398 | 204 |
| 1/11 | 413 | 4543 | 125 |
| 1/21 | 177 | 3717 | 103 |
| 1/31 | 119 | 3689 | 98 |

When the measured values on the specimen at the same dilution ratio of 1/4 were compared, the measured value with the sIL-2R measurement reagent B exhibited a value of about 4-fold as compared with the value measured with the reagent of Example 4. In addition, influence by the nonspecific reaction is almost abolished at a dilution ratio of 1/8 with the reagent of Example 4. In contrast, in case of the sIL-2R measurement reagent B, the nonspecific reaction exhibiting the value of 125% with respect to the theoretical value was found even though the dilution ratio was 1/11, and 1/21 to 1/31 dilution rate was required to almost abolish the influence of the nonspecific reaction. It Is reported that, in general, when an antibody is bound to an enzyme according to a periodic acid method, a high conjugate labeled antibody which is polymerized is produced ("Enzyme Immunoassay Method", 1987, author: Eiji Ishikawa, published by Igaku-Shoin Ltd.). Therefore, the POD-labeled antibody which was labeled according to the periodic acid method used in the sIL-2R measurement reagent B is believed to include antibodies having a high binding number of POD per molecule of the antibody such as the binding ratio between the antibody and POD of 1:4 or 1:5, as well as polymerized antibodies having a binding ratio between the antibody and POD of 2:2, 2:3 and 3:1.

From the foregoings, it was suggested that the reagent of Example 4 in which the POD-labeled antibody in the fractions 40 to 46 in Example 1, i.e., the POD-labeled antibodies having a binding ratio between the antibody and POD of 1:1 and 1:2 were used effectively suppresses also the nonspecific reaction resulting from a factor other than HAMA.

(3) Effect of Inactivated Peroxidase in Nonspecific Reaction in Specimen A

sIL-2R in the 1/4-diluted specimen A and control serum II (obtained by adding sIL-2R to the human normal human serum) was measured with the reagent of Example 4 in which the enzyme-labeled antibody diluent was used to which 200 μg/mL MAK33-IgG Poly or 400 μg/mL Inactive Poly-POD was added. As a control, the measurement was also carried out with the reagent of Example 4. Results are shown in Table 13. sIL2-R included in the control serum II was 2154 U/mL by the measurement with the sIL-2R measurement reagent B, while the theoretical value of sIL2-R in the 1/4-diluted specimen A was calculated to be 906 U/mL (3624 U/mL x 1/4).
[Table 13]

Table 13

| Labeled antibody diluent | sIL-2R measured value (U/mL) | |
| --- | --- | --- |
| | 1/4-diluted specimen A | Control serum II |
| Reagent of Example 4 | 2022 | 2213 |
| + MAK33-IgG Poly | 1034 | 2176 |
| + Inactive Poly-POD | 736 | 2276 |
| sIL-2R theoretical value | 906 | 2154 |

Addition of 200 μg/mL MAK33-IgG Poly or 400 μg/mL inactive Ploy-POD to the reagent of Example 4 did not alter the measured value on the control serum II, therefore, no influence on the measurement system was suggested. The sIL-2R measured value of the 1/4-diluted specimen A with the reagent of Example 4 was 2022 U/mL, suggesting a nonspecific reaction found as compared with the theoretical value. However, by adding MAK33-IgG Poly or Inactive Poly-POD to the labeled antibody diluent, the measurement value was lowered approximately to the theoretical value, showing further suppression of the nonspecific reaction. Particularly, the addition of Inactive Poly-POD almost completely suppressed the nonspecific reaction, therefore, it was revealed that the nonspecific reaction resulting from an antibody that reacts with POD was present. The reagent of Example 4 could suppress the nonspecific reaction in the specimen A, i.e., the nonspecific reaction resulting from the antibody which reacts with POD. Furthermore, by allowing inactivated POD to coexist, the nonspecific reaction resulting from the anti-POD antibody could be sufficiently suppressed.

INDUSTRIAL APPLICABILITY

According to the present invention, a method for immunologically determining an analyte in a sample and a reagent for the method which are useful for monitoring of a pathologic condition and diagnosis of a disease, and a method for suppressing a nonspecific reaction in the method for immunologically determining an analyte are provided.

**Claims**

1. A method for immunologically determining an analyte in a sample, in which a non-specific reaction is suppressed, which comprises the steps of:

   allowing an enzyme-labeled antibody in which an enzyme is bound as a label to a first antibody that specifically binds to the analyte, to react with the sample in an aqueous medium thereby forming an immune complex of the analyte and the enzyme-labeled antibody, said enzyme-labeled antibody substantially containing only one or plural number of enzyme-labeled antibodies selected from the group consisting of enzyme-labeled antibodies having a binding ratio between the first antibody and the enzyme of 1:1, 1:2, 1:3 and 2:1, respectively, and measuring the enzyme activity of the immune complex.

2. The method for immunologically determining an analyte according to claim 1 which further comprises the step of allowing an antibody in which a separation means is bound to a second antibody that specifically binds to the antigenic determination site that is different from the antigenic determination site of the analyte to which the first antibody is bound to react with the sample, thereby forming an immune complex with the analyte.

3. The method for immunologically determining an analyte according to claim 1 or 2 wherein the binding ratio between the first antibody and the enzyme is 1:1, 1:2 and 1:3, respectively.

4. The method for immunologically determining an analyte according to claim 1 or 2 wherein the binding ratio between the first antibody and the enzyme is 1:1 and 1:2, respectively.

5. The method for immunologically determining an analyte according to any one of claims 1 to 4 wherein the first antibody is an antibody in which the Fc region is removed.

6. The method for the immunological determination according to any one of claims 1 to 5 wherein the nonspecific reaction is a reaction that results from a human anti-mouse antibody.

7. The method for immunologically determining an analyte according to any one of claims 1 to 6 wherein an IgG polymer and/or IgG are/is allowed to coexist in the step of allowing the enzyme-labeled antibody to react with the sample, thereby forming the immune complex with the analyte.

8. The method for immunologically determining an analyte according to any one of claims 1 to 5 wherein the nonspecific reaction is a reaction that results from an antibody that reacts with the labeling enzyme.

9. The method for immunologically determining an analyte according to any one of claims 1 to 8 wherein an inactivated enzyme which is the same enzyme as the enzyme labeling the antibody is allowed to coexist in the step of allowing the enzyme-labeled antibody to react with the sample, thereby forming the immune complex, with the analyte.

10. The method for immunologically determining an analyte according to any one of claims 1 to 9 wherein the enzyme is peroxidase.

11. The method for immunologically determining an analyte according to any one of claims 1 to 10 wherein the analyte is a soluble interleukin-2 receptor.

12. A reagent for for immunologically determining an analyte in which a nonspecific reaction is suppressed, which comprises an enzyme-labeled antibody in which an enzyme is bound as a label to a first antibody that specifically binds to the analyte, said enzyme-labeled antibody substantially containing only one or plural number of enzyme-labeled antibodies selected from the group consisting of enzyme-labeled antibodies having a binding ratio between the first antibody and the enzyme of 1:1, 1:2, 1:3 and 2:1, respectively.

13. The reagent according to claim 12 which further includes an antibody in which a separation means is bound to a second antibody that specifically binds to the antigenic determination site that is different from the antigenic determination site of the analyte to which the first antibody is bound.

14. The reagent according to claim 12 or 13 which further comprises a reagent for measuring the enzyme activity.

15. The reagent according to any one of claims 12 to 14 wherein the binding ratio between the first antibody and the enzyme is 1:1, 1:2 and 1:3, respectively.

16. The reagent according to any one of claims 12 to 14 wherein the binding ratio between the first antibody and the enzyme is 1:1 and 1:2, respectively.

17. The reagent according to any one of claims 12 to 16 wherein the first antibody is an antibody in which the Fc region is removed.

18. The reagent according to any one of claims 12 to 17 wherein the nonspecific reaction is a reaction that results from a human anti-mouse antibody.

19. The reagent according to any one of claims 12 to 18 which further comprises an IgG polymer and/or IgG.

20. The reagent according to any one of claims 12 to 17 wherein the nonspecific reaction is a reaction that results from an antibody that reacts with the labeling enzyme.

21. The reagent according to any one of claims 12 to 20 which further comprises an inactivated enzyme which is the same enzyme as the enzyme labeling the antibody.

22. The reagent according to any one of claims 12 to 21 wherein the enzyme is peroxidase.

23. The reagent according to any one of claims 12 to 22 wherein the analyte is a soluble interleukin-2 receptor.

24. The reagent according to any one of claims 12 to 23 which further comprises one or plural number of substances selected from the group consisting of an aqueous medium, a metal ion, a salt, saccharide, a surfactant, an antiseptic agent, protein and a protein stabilizing agent.

25. A method for suppressing a nonspecific reaction in a method for immunologically determining an analyte, which comprises the step of:

allowing an enzyme-labeled antibody in which an enzyme is bound as a label to a first antibody that specifically binds to the analyte, to react with the sample in an aqueous medium thereby forming an immune complex of the analyte and the enzyme-labeled antibody, said enzyme-labeled antibody substantially containing only one or plural number of enzyme-labeled antibodies selected from the group consisting of enzyme-labeled antibodies having a binding ratio between the first antibody and the enzyme of 1:1, 1:2, 1:3 and 2:1, respectively.

26. The method for suppressing a nonspecific reaction according to claim 25 which further comprises the step of:

allowing an antibody in which a separation means is bound to a second antibody that specifically binds to the antigenic determination site that is different from the antigenic determination site of the analyte to which the first antibody is bound to react with the sample, thereby forming an immune complex with the analyte.

27. The method for the suppression according to claim 25 or 26 wherein the binding ratio between the first antibody and the enzyme is 1:1, 1:2 and 1:3, respectively.

28. The method for suppressing a nonspecific reaction according to claim 25 or 26 wherein the binding ratio between the first antibody and the enzyme is 1:1 and 1:2, respectively.

29. The method for suppression a nonspecific reaction according to any one of claims 25 to 28 wherein the first antibody is an antibody in which the Fc region is removed.

30. The method for suppression a nonspecific reaction according to any one of claims 25 to 29 wherein the nonspecific reaction is a reaction that results from a human anti-mouse antibody.

31. The method for suppressing a nonspecific reaction according to any one of claims 25 to 30 wherein an IgG polymer and/or IgG are/is allowed to coexist in the step of allowing the enzyme-labeled antibody to react with the sample, thereby forming the immune complex with the analyte.

32. The method for suppressing a nonspecific reaction according to any one of claims 25 to 29 wherein the nonspecific reaction is a reaction that results from an antibody that reacts with the labeling enzyme.

33. The method for suppressing a nonspecific reaction according to any one of claims 25 to 32 wherein an in activated enzyme which is the same enzyme as the enzyme labeling the antibody is allowed to coexist in the step of allowing the enzyme-labeled antibody to react with the sample, thereby forming the immune complex with the analyte.

34. The method for suppressing a nonspecific reaction according to any one of claims 25 to 33 wherein the enzyme is peroxidase.

35. The method for suppressing a nonspecific reaction according to any one of claims 25 to 34 wherein the analyte is a soluble interleukin-2 receptor.

Fig. 1

<div align="center">

### INTERNATIONAL SEARCH REPORT

</div>

| | International application No. |
|---|---|
| | PCT/JP2005/010882 |

**A. CLASSIFICATION OF SUBJECT MATTER**
Int.Cl$^7$ G01N33/543, 33/53, 33/531

According to International Patent Classification (IPC) or to both national classification and IPC

**B. FIELDS SEARCHED**

Minimum documentation searched (classification system followed by classification symbols)
Int.Cl$^7$ G01N33/543, 33/53, 33/531

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2005 |
| Kokai Jitsuyo Shinan Koho | 1971-2005 | Toroku Jitsuyo Shinan Koho | 1994-2005 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | JP 2001-183375 A (Tosoh Corp.), 06 July, 2001 (06.07.01), Claims 1, 4; Par. Nos. [0004], [0008], [0016] | 1-6,8,10, 12-18,20,22, 24-30,32,34 |
| Y | (Family: none) | 7,9,11,19, 21,23,31,33, 35 |
| Y | JP 8-23560 B2 (Boehringer Mannheim GmbH.), 06 March, 1996 (06.03.96), Claim 6; page 2, right column, lines 16 to 37 & EP 0331068 A & US 4914040 A | 7,19,31 |
| Y | JP 5-188055 A (Takeda Chemical Industries, Ltd.), 27 July, 1993 (27.07.93), Claims 1, 2 (Family: none) | 9,21,33 |

| ☒ Further documents are listed in the continuation of Box C. | ☐ See patent family annex. |
|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 11 July, 2005 (11.07.05) | 26 July, 2005 (26.07.05) |

| Name and mailing address of the ISA/ Japanese Patent Office | Authorized officer |
|---|---|
| Facsimile No. | Telephone No. |

Form PCT/ISA/210 (second sheet) (January 2004)

# EP 1 767 942 A1

**INTERNATIONAL SEARCH REPORT**

International application No.

PCT/JP2005/010882

C (Continuation). DOCUMENTS CONSIDERED TO BE RELEVANT

| Category* | Citation of document, with indication, where appropriate, of the relevant passages | Relevant to claim No. |
|---|---|---|
| Y | JP 62-70761 A (The United States of America), 01 April, 1987 (01.04.87), Claims (1), (2), (5) & EP 0202975 A & US 4707443 A | 11,23,35 |

Form PCT/ISA/210 (continuation of second sheet) (January 2004)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 61065162 A
- JP 1254869 A
- JP 5188055 A
- JP 62070761 A

**Non-patent literature cited in the description**

- *Clinical Chemistry,* 1999, vol. 45, 942-956
- *Cancer Immunological Immunotherapy,* 1991, vol. 33, 80-84
- *Clinical Chemistry,* 1990, vol. 36, 1093
- **EIJI ISHIKAWA.** Enzyme Immunoassay Method. Igaku-Shoin Ltd, 1987
- *J. Immunol.,* 1985, vol. 135, 3172-3177